# EUROPEAN PATENT APPLICATION

(11) **EP 4 803 633 A1**
(43) Date of publication of application: **09.09.2026**
(21) Application number: 26162369.8
(22) Date of filing: 04.03.2026
(51) Int. Cl.: C12Q 1/04, C12Q 1/24, C12N 1/00, C12M 1/12, C12M 1/34, C12Q 1/689

(54) **METHOD, SYSTEM, AND KIT FOR DETECTING MICROORGANISMS**

(30) Priority: 05.03.2025 JP 2025034931
(71) Applicant: Yokogawa Electric Corporation, Musashino-shi, Tokyo 180-8750 (JP)
(72) Inventor: MAEMURA, Chika, Musashino-shi, Tokyo, 180-8750 (JP); TAGUCHI, Tomoyuki, Musashino-shi, Tokyo, 180-8750 (JP)
(74) Representative: Stöckeler, Ferdinand

(57) **Abstract**

A method for detecting microorganisms is provided in which when target microorganisms are detected in a microorganism-containing sample, ease of handling like liquid culture media is maintained, and even when multiple bacterial species are present in the sample, each bacterial species is allowed to grow sufficiently by suppressing growth inhibition by the influence of other bacterial species, thereby improving the detection accuracy of the target microorganisms. The method includes: a) inoculating a microorganism-containing sample into a culture medium for microorganisms that contains an environment-responsive polymer that can cause the solution to thermoreversibly transition between fluidized and non-fluidized states depending on a solution environment; b) culturing microorganisms while maintaining the culture medium in a non-fluidizing environment; c) adjusting the culture medium to a fluidizing environment after the culturing of the microorganisms; d) recovering the cultured microorganisms from the culture medium; and e) detecting target microorganisms in the recovered microorganisms.

## Description

### CROSS-REFERENCE TO RELATED APPLICATION

This application claims priority to Japanese Patent Application No. 2025-034931, filed on March 5, 2025, the entire contents of which are incorporated herein by reference.

### TECHNICAL FIELD

The present disclosure relates to a method, system, and kit for detecting microorganisms.

### BACKGROUND

The detection of target microorganisms in microorganism-containing samples, such as body-derived samples and environmental materials, in which multiple bacterial species are present is used for health condition management, environmental analyses, and the like. For example, Patent Literature (PTL) 1 describes a method for isolating and culturing specific microorganisms by culturing a microbial community in a liquid culture medium and culturing the microbial community in the presence of a specific antibiotic during a specific growth period. PTL 2 describes an assay for identifying unknown microorganisms in a mixed culture by culturing the microorganisms in an aqueous medium containing a nucleic acid analog and capturing nucleic acids incorporating the analog using an analog-specific binding member.

PTL 3 describes a microbial culture method for growing a target bacterium contained in an antibacterial test sample, using a microbial culture medium characterized in that a basal medium for microbial culture contains activated clay and activated carbon in the microbial culture medium for growing the target bacterium contained in the antibacterial test sample.

### CITATION LIST

### Patent Literatures

PTL 1: JP 2015-188407 A
PTL 2: JP 2002-509731 A
PTL 3: JP 5464465 B2

### SUMMARY

For example, as illustrated in FIGS. 4 and 5, when a microorganism-containing sample with multiple bacterial species present is cultured in a liquid culture medium for microorganism detection, while the handling becomes easier, bacterial growth may be inhibited due to differences in growth rates among the bacterial species and interactions between the different bacterial species. The growth of a target bacterial species may be reduced compared to when a single bacterial species is cultured, and the amount of nucleic acids extracted in nucleic acid detection may be significantly reduced and become undetectable. On the other hand, as illustrated in FIGS. 6 and 7, when a membrane filter with a microorganism-containing sample immersed is placed on a solid culture medium and multiple bacterial species are cultured on the membrane filter, bacterial growth inhibition, which is caused by differences in growth rates and interactions between different bacterial species, is unlikely to occur owing to the distances between the bacteria. However, it is difficult to separate the membrane filter from the bacteria for analysis, thus resulting in difficulty in handling.

The present disclosure aims to provide a method for detecting microorganisms in which when target microorganisms are detected in a microorganism-containing sample, ease of handling like liquid culture media is maintained, and even when multiple bacterial species are present in the sample, each bacterial species is allowed to grow sufficiently by suppressing growth inhibition by the influence of other bacterial species, thereby improving the detection accuracy of the target microorganisms. Furthermore, the present disclosure aims to provide a system and kit for detecting microorganisms that can be used for this method.

The inventors have found out that a microorganism-containing sample in the form of a sheet substrate such as a membrane filter is immersed in a culture medium in a fluidized state, using a thermosensitive polymer-containing culture medium for microorganisms that can be adjusted in a thermoreversible manner between fluidized and non-fluidized states depending on temperature, is cultured while the culture medium is maintained in a non-fluidized state, and is recovered after the cultivation by making the culture medium in the fluidized state. Thereby, the positions of bacterial species are fixed during the cultivation and the distances between the bacterial species are maintained. This suppresses bacterial growth inhibition due to differences in growth rates and interactions between the bacterial species and allows each bacterial species to grow sufficiently, thus improving the detection accuracy of target microorganisms.

The present disclosure is as follows.
[1]
   A method for detecting microorganisms including:
   a) inoculating a microorganism-containing sample into a culture medium for microorganisms that contains an environment-responsive polymer that can cause the solution to transition in a thermoreversible manner between a fluidized state and a non-fluidized state depending on a solution environment;
   b) culturing microorganisms while maintaining the culture medium in a non-fluidizing environment;
   c) adjusting the culture medium to a fluidizing environment after the culturing of the microorganisms;
   d) recovering the cultured microorganisms from the culture medium; and
   e) detecting target microorganisms in the recovered microorganisms.
[2]
   The method described in [1], wherein
   the environment-responsive polymer is a thermosensitive polymer,
   the culture medium for microorganisms is brought into the fluidized state in a thermoreversible manner at temperatures below a transition temperature, and is brought into the non-fluidized state in a thermoreversible manner at temperatures above the transition temperature,
   the step b) is performed by maintaining the culture medium at a temperature at which the culture medium becomes non-fluidized, and
   the step c) is performed by adjusting the culture medium to a temperature at which the culture medium becomes fluidized.
[3]
   The method described in [2], wherein the step a) is performed by immersing a solid substrate on which the microorganisms are captured, in the culture medium that has been adjusted to a temperature at which the culture medium becomes fluidized.
[4]
   The method described in [1] or [2], wherein the step e) is performed by detecting nucleic acids, proteins, or components other than nucleic acids and proteins specific to the target microorganisms in the recovered microorganisms.
[5]
   The method described in [3], wherein the step e) is performed by:
   extracting nucleic acids from the recovered microorganisms; and
   detecting a nucleotide sequence specific to the target microorganisms in the extracted nucleic acids.
[6]
   The method described in [2], wherein
   the step b) is performed at a temperature at least 1°C and at most 40°C above the transition temperature, and
   the step c) is performed at a temperature at least 1°C and at most 40°C below the transition temperature.
[7]
   The method described in [2], wherein
   the step b) is performed at a temperature of 10°C or higher and 60°C or lower, and
   the step c) is performed at a temperature of 1°C or higher and 40°C or lower.
[8]
   The method described in [2], wherein the transition temperature is 1°C or higher and 40°C or lower.
[9]
   The method described in [2], wherein the thermosensitive polymer is readily water-soluble in a thermoreversible manner at temperatures below the transition temperature, and is poorly water-soluble in a thermoreversible manner at temperatures above the transition temperature.
[10]
   The method described in [8], wherein the environment-responsive polymer is:
   a thermosensitive polymer selected from a group consisting of any one of poly(N-isopropylacrylamide) (PNIPAM), polyacrylamide, polymethacrylate, polycyclodextran, synthetic elastin polymers, amphiphilic polymers with multidentate chelating ability, hydrogels, hyaluronic acid complexes, natural hydrogels, synthetic hydrogels, silicone, polyurethane, polysulfone, cellulose, polyethylene, polypropylene, polyamide, polyimide, polyester, polytetrafluoroethylene, polyvinyl chloride, epoxy resin, phenolic resin, neoprene, and polyisoprene, as well as any combination thereof; or
   a pH-sensitive polymer selected from a group consisting of any one of copolymers of N-isopropylacrylamide (NIPAM) and acrylic acid, propylacrylic acid, or 2-carboxyisopropylacrylamide, as well as any combination thereof.
[11]
   A system for detecting microorganisms including:
   A) a culture medium environment adjuster configured to:
      adjust an environment of a culture medium for microorganisms containing an environment-responsive polymer to a fluidizing environment when a sample containing microorganisms is inoculated;
      maintain the environment of the culture medium in a non-fluidizing environment while the microorganisms are being cultured; and
      adjust the environment of the culture medium to a fluidizing environment when the cultured microorganisms are recovered;
   B) a microorganism recoverer configured to recover the cultured microorganisms from the culture medium; and
   C) a nucleic acid extractor configured to extract nucleic acids from the recovered microorganisms.
[12]
   The system described in [11], wherein
   A) the culture medium environment adjuster is configured to:
   adjust a temperature of a culture medium for microorganisms containing a thermosensitive polymer to a fluidizing temperature when a sample containing microorganisms is inoculated;
   maintain the temperature of the culture medium at a non-fluidizing temperature while the microorganisms are being cultured; and
   adjust the temperature of the culture medium to a fluidizing temperature when the cultured microorganisms are recovered.
[13]
   The system described in [11] or [12], further including:
   D) a nucleic acid detector configured to detect a nucleotide sequence specific to target microorganisms in the nucleic acids extracted from the microorganisms.
[14]
   A kit for detecting microorganisms, including:
   i) the system described in any one of [11] to [13]; and
   ii) any one of the following ii-1) to ii-3):
      ii-1) an environment-responsive polymer that, when dissolved in a culture medium for microorganisms, can cause the solution to transition in a thermoreversible manner between a fluidized state and a non-fluidized state depending on a solution environment;
      ii-2) a culture medium component composition containing a thermosensitive polymer and a culture medium component for microorganisms; or
      ii-3) a culture medium for microorganisms containing a thermosensitive polymer.
[15]
   The kit described in [14], wherein the environment-responsive polymer is a thermosensitive polymer.
[16]
   The kit described in [14] or [15], further including at least one or more of the following:
   iii) a sheet substrate for capturing microorganisms;
   iv) a culture vessel capable of being installed in the system, the culture vessel containing the culture medium and the sheet substrate; and
   v) a probe or primer for detecting a nucleotide sequence specific to target microorganisms.

According to the present disclosure, when target microorganisms are detected in a microorganism-containing sample, ease of handling like liquid culture media is maintained, and even when multiple bacterial species are present in the sample, each bacterial species is allowed to grow sufficiently by suppressing growth inhibition due to the influence of other bacterial species, thereby improving the detection accuracy of the target microorganisms.

### BRIEF DESCRIPTION OF THE DRAWINGS

In the accompanying drawings:

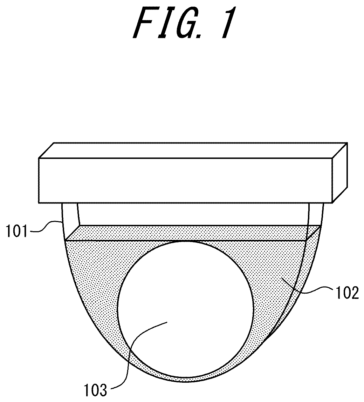

FIG. 1 is a schematic diagram illustrating an example of a microorganism culture system to perform a method for detecting microorganisms according to the present disclosure;

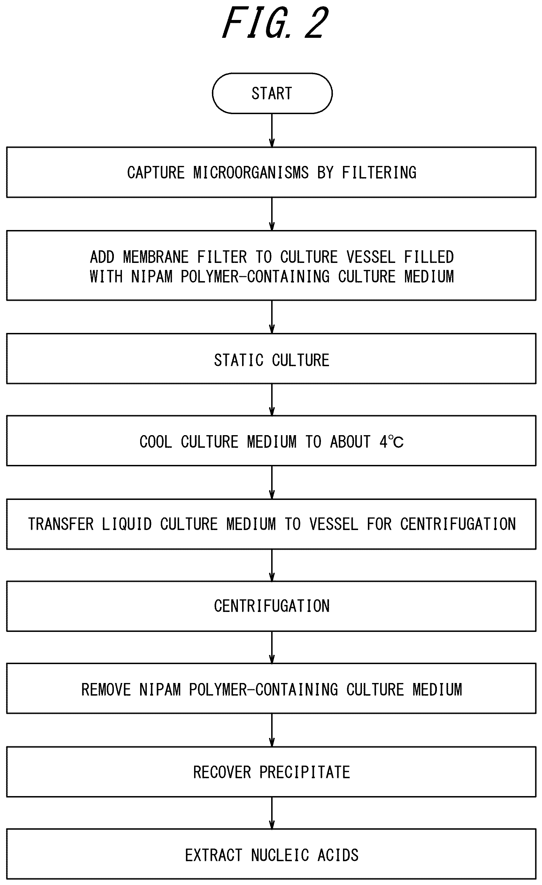

FIG. 2 illustrates an example of a procedure of the method for detecting microorganisms according to the present disclosure;

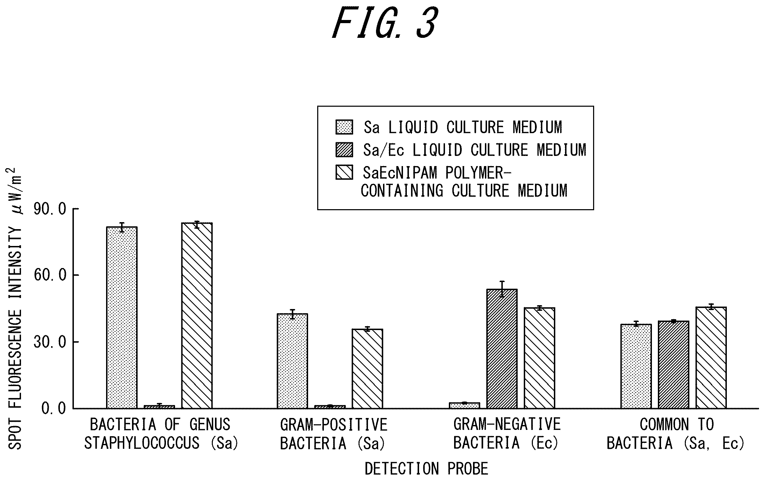

FIG. 3 illustrates an example of the results of detection of target microorganisms by the method for detecting microorganisms according to the present disclosure;

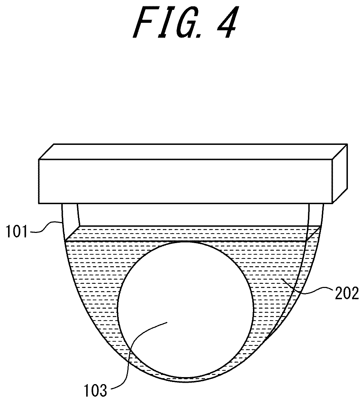

FIG. 4 is a schematic diagram illustrating an example of a microorganism culture system to perform a conventional method for detecting microorganisms using a liquid culture medium;

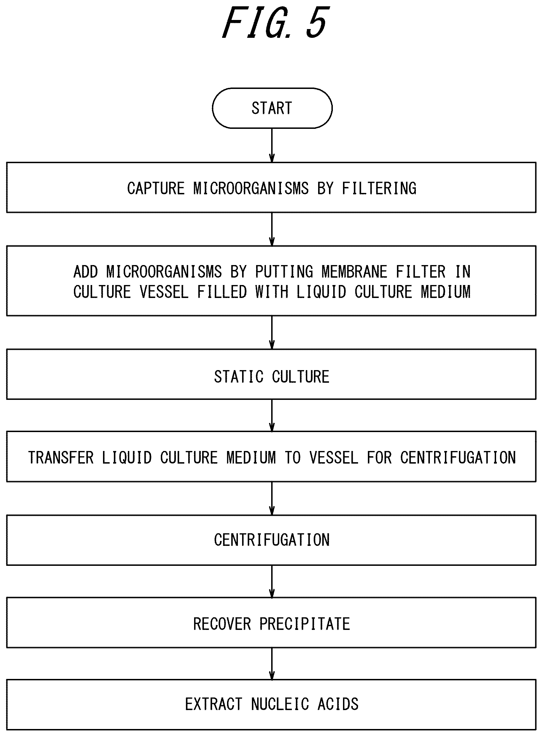

FIG. 5 illustrates an example of a procedure of the conventional method for detecting microorganisms using the liquid culture medium;

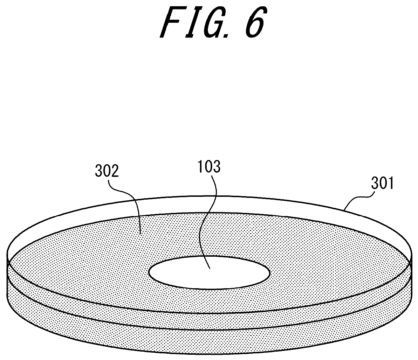

FIG. 6 is a schematic diagram illustrating an example of a microorganism culture system to perform a conventional method for detecting microorganisms using a solid culture medium; and

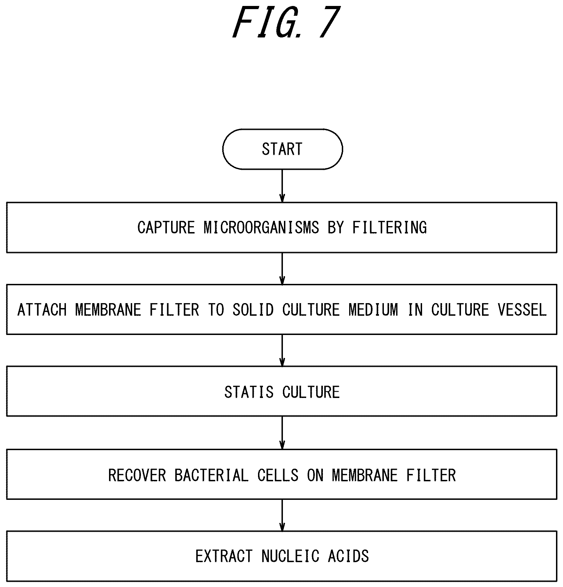

FIG. 7 illustrates an example of a procedure of the conventional method for detecting microorganisms using the solid culture medium.

### DETAILED DESCRIPTION

The present disclosure will be described in detail below with reference to the drawings as necessary. The drawings are examples to illustrate the present disclosure, and the technical scope of the present disclosure is not limited by the examples provided by the drawings.

### [Method for Detecting Microorganisms]

A method for detecting microorganisms of the present disclosure includes the following steps:
a) inoculating a microorganism-containing sample into a culture medium for microorganisms that contains an environment-responsive polymer that can cause the solution to transition in a thermoreversible manner between a fluidized state and a non-fluidized state depending on a solution environment;
b) culturing microorganisms while maintaining the culture medium in a non-fluidizing environment;
c) adjusting the culture medium to a fluidizing environment after the culturing of the microorganisms;
d) recovering the cultured microorganisms from the culture medium; and
e) detecting target microorganisms in the recovered microorganisms.

### (Microorganisms)

In the present disclosure, "microorganisms" are used to refer to minute organisms that are difficult to observe with the naked eye, and mainly refer to organisms that are 1 mm or less in length. Organism species are not particularly limited. Examples of microorganisms include eukaryotes such as microalgae, protozoa, fungi, and slime molds, and prokaryotes such as eubacteria and archaebacteria. The types of microorganisms include, for example, microalgae, yeasts, eubacteria, and filamentous cells. Examples of microalgae include the genera Tetraselmis, Nannochloropsis, Dunaliella, Phaeodactylum, Isochrysis, Chlorella, Haematococcus, Spirulina, Scenedesmus, Chlamydomonas, and others. Examples of yeasts include the genera Saccharomyces, Candida, Pichia, Kluyveromyces, Zygosaccharomyces, Schizosaccharomyces, Debaryomyces, Hansenula, Torulopsis, Yarrowia, and others. Examples of eubacteria include gram-negative bacilli such as the genera Escherichia, Pseudomonas, Sphingomonas, and Methylobacterium, and gram-positive bacteria such as the genera Staphylococcus and Bacillus. Examples of filamentous fungi include the genera Aspergillus, Penicillium, Rhizopus, Fusarium, Trichoderma, Mucor, Neurospora, Alternaria, Beauveria, Cladosporium, and others.

### (Culture Medium for Microorganisms)

As culture media for microalgae, appropriate media can be used, depending on the type of strain used. Such culture media can be used alone or in combination depending on whether the target microalgae are mainly found in freshwater or saltwater. Examples of culture media for freshwater microalgae include BG11 media, Z8 media, ASM-11 media, TAP media, CHU-10 media, WC media, BBM media, AAP media, and the like. Examples of culture media for seawater microalgae include f/2 media, ESM media, MNK media, and the like.

As culture media for Escherichia coli, various culture media can be used, depending on the type of strain used. Examples of such culture media include LB media, TSA media, NA media, MacConkey media, EMB media, M9 minimal media, SOB media, TB media, SCD media, and the like, and can be used alone or in combination.

As culture media for yeasts, various culture media can be used, depending on the type of strain used. Examples of such culture media include YPD media, YPG media, SD media, SGal media, PDA media, SC media, YMM media, YM media, and the like, and can be used alone or in combination.

As culture media for filamentous fungi, various culture media can be used, depending on the type of strain used. Examples of such culture media include PDA media, SDA media, Czapek-Dox media, MEA media, CMA media, V8 Juice Agar media, YES media, OA media, Czapek yeast extract media, Emerson YpSs media, and the like, and can be used alone or in combination.

### (Environment-responsive Polymer)

In the present disclosure, "environment-responsive polymer" refers to a polymer that, when dissolved in water or an aqueous solution (especially, a culture medium for microorganisms), causes the solution to transition in a thermoreversible manner between a fluidized state and a non-fluidized state depending on a solution environment or stimulus. The fluidized state is a state in which solation of the solution occurs primarily due to the environment-responsive polymer being readily water-soluble (hydrophilic). The non-fluidized state is a state in which gelation of the solution occurs primarily due to the environment-responsive polymer being poorly water-soluble (hydrophobic). Examples of "environment-responsive polymer" include thermosensitive polymers, pH-sensitive polymers, and photoresponsive polymers. "Thermosensitive polymers" refer to polymers that, when dissolved in water or an aqueous solution, cause the solution to become fluidized in a thermoreversible manner at temperatures below a transition temperature, and to become non-fluidized at temperatures above the transition temperature. As the thermosensitive polymers, polymers that are readily water-soluble (e.g., cause the water or aqueous solution to undergo solation) in a thermoreversible manner at temperatures below the transition temperature, and that are poorly water-soluble (e.g., cause the water or aqueous solution to undergo gelation) at temperatures above the transition temperature are used. The transition temperature is not particularly limited, but should be a temperature that is higher than the freezing point of water and that satisfies the conditions that allow microorganisms to be cultured at temperatures above the transition temperature. The transition temperature may be, for example, 1°C or higher, preferably 4°C or higher, more preferably 10°C or higher, and may be, for example, 40°C or lower, preferably 20°C or lower, more preferably 15°C or lower. "pH-sensitive polymers" refer to polymers that, when dissolved in water or an aqueous solution, cause the solution to become fluidized in a reversible manner at pHs below or above a transition pH, and to become non-fluidized at pHs above or below the transition pH. The transition pH is not particularly limited, but should be a pH that does not overload microorganisms. The transition pH may be, for example, 3 or higher, preferably 5 or higher, more preferably 7 or higher, and may be, for example, 10 or lower, preferably 9 or lower, more preferably 8 or lower. "Photoresponsive polymers" refer to polymers that, when dissolved in water or an aqueous solution, cause the solution to transition in a thermoreversible manner between a fluidized state and a non-fluidized state, dependent on light irradiation or light interception.

Examples of thermosensitive polymers include poly(N-isopropylacrylamide) (PNIPAM) (polymers obtained by homopolymerization of N-isopropylacrylamide (NIPAM)), polyacrylamide, polymethacrylate, polycyclodextran, synthetic elastin polymers, amphiphilic polymers with multidentate chelating ability, hydrogels, hyaluronic acid complexes, natural hydrogels, synthetic hydrogels, silicone, polyurethane, polysulfone, cellulose, polyethylene, polypropylene, polyamide, polyimide, polyester, polytetrafluoroethylene, polyvinyl chloride, epoxy resins, phenolic resins, neoprene, polyisoprene, or any combination thereof. The transition temperatures of these polymers can be adjusted as appropriate by changing the degree of polymerization (weight-average molecular weight and number-average molecular weight). For example, in the case of PNIPAM, the transition temperature can be set around 30°C by setting the weight-average molecular weight from about 10,000 to about 110,000. In a culture medium for microorganisms that contains a thermosensitive polymer (hereinafter abbreviated as "thermosensitive culture medium"), the concentration of the thermosensitive polymer may be, for example, 2 g/L or more, preferably 5 g/L or more, more preferably 10 g/L or more, and may be, for example, 200 g/L or less, preferably 50 g/L or less, more preferably 10 g/L or less. Examples of pH-sensitive polymers include copolymers of N-isopropylacrylamide (NIPAM) and acrylic acid, propylacrylic acid, or 2-carboxyisopropylacrylamide, and the like. Examples of photoresponsive polymers include polymeric spiropyrans and the like.

### (Culture Vessel)

Throughout the steps a) to c), the thermosensitive culture medium is usually contained in a culture vessel. The culture vessel is not limited to any particular shape as long as the culture vessel can accommodate the culture medium in the fluidized state and a solid substrate (especially a sheet substrate) described below. It is preferable that the shape should allow for easy temperature adjustment from outside. Examples of such shapes include vertically flattened shapes, as illustrated in FIG. 1.

### (Step a): Inoculating Microorganisms)

The step a) is usually performed at a temperature at which the thermosensitive culture medium is fluidized, that is, by bringing the thermosensitive culture medium into the fluidized state (e.g., sol state). The temperature at which the thermosensitive culture medium is fluidized may be the same as a temperature at which the step c), described below, is performed. The microorganism-containing sample may be a solid substrate on which microorganisms are captured or a solution (e.g., culture medium) containing microorganisms. However, the solid substrate on which microorganisms are captured is preferable from the viewpoint of facilitating the fixation of the positions of microorganisms to be cultured in the step b) and suppressing growth inhibition by the effects of other microorganisms. Examples of solid substrates include sheet substrates, particle substrates, and the like. Among these, sheet substrates are preferable from the viewpoint of ease of handling. Examples of sheet substrates include membrane filters (e.g., made of cellulose resin, nylon, fluorocarbon resin, polycarbonate resin, or sulfone resin) and the like. When a solid substrate is used as a microorganism-containing sample, the step a) can be performed by immersing the solid substrate on which microorganisms are captured in a culture medium adjusted to a temperature at which the culture medium becomes fluidized. When a sheet substrate is used as a microorganism-containing sample, microorganisms can be captured on the sheet substrate by immersing the sheet substrate in a solution containing microorganisms or filtering a solution containing microorganisms by the sheet substrate.

### (Step b): Culturing Microorganisms)

The step b) is performed by maintaining the culture medium in the non-fluidizing environment, that is, by bringing the thermosensitive culture medium into the non-fluidized state (e.g., gel state). When the environment-responsive polymer is a thermosensitive polymer, the temperature at which the step b) is performed is a temperature at which each microorganism grows while making the culture medium non-fluidized. In the step b), since each microorganism grows in the culture medium in the non-fluidized state, the position of each bacterial cell is fixed during the cultivation. Maintaining the distances between bacterial cells suppresses bacterial growth inhibition due to differences in growth rates and interactions between bacterial species, and allows each bacterial species to grow sufficiently, thus improving the detection accuracy of the target microorganisms. The step b) may be performed, for example, at a temperature 1°C or more above the transition temperature, preferably 10°C or more above the transition temperature, and more preferably 40°C or more above the transition temperature. The step b) may also be performed at a temperature of, for example, 10°C or higher, preferably 30°C or higher, more preferably 35°C or higher, and, for example, 60°C or lower, preferably 45°C or lower, more preferably 40°C or lower.

### (Step c): Fluidizing Culture Medium)

The step c) is performed by adjusting the culture medium to the fluidizing environment, that is, by bringing the thermosensitive culture medium into the fluidized state (e.g., sol state). When the environment-responsive polymer is a thermosensitive polymer, the step c) may be performed at a temperature of, for example, 1°C or more below the transition temperature, preferably 10°C or more below the transition temperature, and more preferably 40°C or more below the transition temperature. The step c) may also be performed at a temperature of, for example, 1°C or higher, preferably 2°C or higher, more preferably 4°C or higher, and, for example, 40°C or lower, preferably 10°C or lower, more preferably 4°C or lower.

### (Step d): Recovering Microorganisms)

The step d) may be performed, for example, by transferring the culture medium after culturing the microorganisms, which has brought into the fluidized state, from the culture vessel to a recovery vessel, and then subjecting the culture medium in the recovery vessel to centrifugation, filtering, or the like to recover the microorganisms from the culture medium.

### (Step e): Detecting Microorganisms)

The step e) may be performed by detecting nucleic acids, proteins, or components other than nucleic acids and proteins specific to the target microorganisms in the recovered microorganisms. Here, "nucleic acids specific to the target microorganisms" mean, in particular, nucleic acids (DNA or RNA) that contain a nucleotide sequence specific to the target microorganisms. "Detecting the nucleic acids specific to the target microorganisms" may be considered synonymous with "detecting the nucleotide sequence specific to the target microorganisms in the recovered nucleic acids (DNA or RNA) of the microorganisms". Examples of "components other than nucleic acids and proteins" include cell membrane or cell wall components (e.g., phospholipids, glycolipids, sterols, and other lipids). The detection may be performed by extracting nucleic acids, proteins, or components other than these from the recovered microorganisms and detecting nucleic acids, proteins, or components other than these specific to the target microorganisms in the extract, or by detecting nucleic acids, proteins, or components other than these specific to the target microorganisms by staining in the recovered microorganisms. As a simplified method, the step e) is preferably performed by extracting nucleic acids from the recovered microorganisms and detecting a nucleotide sequence specific to the target microorganisms in the extracted nucleic acids. Examples of nucleotide sequence detection methods include hybridization using probes, and PCR or RT-PCR (including quantitative PCR) using primers, sequencing (e.g., next-generation sequencing), and the like. Examples of protein (including glycoprotein and peptidoglycan) detection methods include immunoblotting, immunological staining, Gram staining, and the like.

FIG. 1 is a schematic diagram illustrating an example of a microorganism culture system to perform the method for detecting microorganisms according to the present disclosure. FIG. 2 illustrates an example of a procedure of the method for detecting microorganisms according to the present disclosure.

### [System for Detecting Microorganisms]

A system for detecting microorganisms according to the present disclosure can be used for the method for detecting microorganisms according to the present disclosure and includes the following:
A) a culture medium environment adjuster configured to:
   adjust an environment of a culture medium for microorganisms containing an environment-responsive polymer to a fluidizing environment when a sample containing microorganisms is inoculated;
   maintain the environment of the culture medium in a non-fluidizing environment while the microorganisms are being cultured; and
   adjust the environment of the culture medium to a fluidizing environment when the cultured microorganisms are recovered;
B) a microorganism recoverer configured to recover the cultured microorganisms from the culture medium; and
C) a nucleic acid extractor configured to extract nucleic acids from the recovered microorganisms.

A) The culture medium environment adjuster may be configured to:
adjust the temperature of a culture medium for microorganisms containing a thermosensitive polymer to a fluidizing temperature when a sample containing microorganisms is inoculated;
maintain the temperature of the culture medium at a non-fluidizing temperature while the microorganisms are being cultured; and
adjust the temperature of the culture medium to a fluidizing temperature when the cultured microorganisms are recovered.

The system for detecting microorganisms according to the present disclosure may further include:
D) a nucleic acid detector configured to detect a nucleotide sequence specific to target microorganisms in the nucleic acids extracted from the microorganisms.

### (Component A): Culture Medium Environment Adjuster)

The culture medium environment adjuster is a component for adjusting the temperature of a thermosensitive culture medium so as to adjust the thermosensitive culture medium to a fluidizing temperature to perform the step a), maintain the temperature of the thermosensitive culture medium at a non-fluidizing temperature to perform the step b), and adjust the temperature of the thermosensitive culture medium to a fluidizing temperature to perform the step c) in the method for detecting microorganisms according to the present disclosure. The culture medium environment adjuster may include a heating/cooling unit for heating or cooling the culture medium or a culture vessel containing the culture medium, a temperature sensor for measuring the temperature of the culture medium or the culture vessel containing the culture medium, and a temperature controller for controlling the heating or cooling by the heating/cooling unit according to the temperature measured by the temperature sensor or a time schedule of the steps a) to c). As temperature conditions to be applied to the culture medium environment adjuster, the conditions described in the method for detecting microorganisms according to the present disclosure can be used.

### (Component B): Microorganism Recoverer)

The microorganism recoverer is a component for performing the step d) in the method for detecting microorganisms according to the present disclosure. The microorganism recoverer may include any one or more of a recovery vessel to which the culture medium after culturing the microorganisms, which has brought into the fluidized state, is transferred from the culture vessel, or a centrifuge or filtering device for subjecting the culture medium in the recovery vessel to centrifugation, filtering, or the like to recover the microorganisms from the culture medium.

### (Component C): Nucleic Acid Extractor)

The nucleic acid extractor is a component for extracting nucleic acids from the recovered microorganisms as part of the step e) in the method for detecting microorganisms according to the present disclosure. The nucleic acid detector may be a series of devices for performing a general nucleic acid extraction method (e.g., nucleic acid extraction method using a chaotropic agent, a filter, and the like).

### (Component D): Nucleic Acid Detector)

The nucleic acid detector is a component for detecting a nucleotide sequence specific to target microorganisms in the extracted nucleic acids as part of the step e) in the method for detecting microorganisms according to the present disclosure. Examples of nucleotide sequence detectors include series of devices for performing hybridization, PCR or RT-PCR (including quantitative PCR), sequencing (e.g., next-generation sequencing), and the like.

### [Kit for Detecting Microorganisms]

A kit for detecting microorganisms according to the present disclosure can be used for the method for detecting microorganisms according to the present disclosure and includes at least one or more of the following:
i) the above-described system for detecting microorganisms; and
ii) any one of the following ii-1) to ii-3):
   ii-1) an environment-responsive polymer that, when dissolved in a culture medium for microorganisms, can cause the solution to transition in a thermoreversible manner between a fluidized state and a non-fluidized state depending on a solution environment;
   ii-2) a culture medium component composition containing an environment-responsive polymer and a culture medium component for microorganisms; or
   ii-3) a culture medium for microorganisms containing an environment-responsive polymer.

The kit for detecting microorganisms according to the present disclosure may further include at least one or more of the following:
iii) a sheet substrate for capturing microorganisms;
iv) a culture vessel capable of being installed in the system, the culture vessel containing the culture medium and the sheet substrate; and
v) a probe or primer for detecting a nucleotide sequence specific to target microorganisms.

Preferably, the kit for detecting microorganisms according to the present disclosure includes at least i) or ii) above. "Culture medium component for microorganisms" in ii-2) above refers to a mixture of components that constitute a culture medium for microorganisms when dissolved in water. The above ii-1) and ii-2) can be used as raw materials for preparing environment-responsive culture media in the method for detecting microorganisms according to the present disclosure. Examples of environment-responsive polymers include thermosensitive polymers, pH-sensitive polymers, photoresponsive polymers, and the like, as described above.

### EXAMPLES

The present disclosure will be described in more detail below using examples, but the technical scope of the present disclosure is not limited by these examples.

FIG. 3 illustrates fluorescence intensity when nucleic acid extraction was performed according to the protocol of the microorganism contamination test, and the extracted nucleic acids were hybridized with probes for the presence of multiple bacterial species (Escherichia coli (Ec) and Staphylococcus aureus (Sa)). When Ec and Sa were cultured simultaneously in a liquid culture medium, the fluorescence intensity of a probe for detecting bacteria of the genus Staphylococcus and a probe for detecting Gram-positive bacteria, which are targets for detection of Sa, was low and undetectable. This is considered to be caused by growth inhibition of Sa occurred and Sa was not cultured sufficiently owing to simultaneous culture with Ec, because when only Sa was cultured in a liquid culture medium, the fluorescence intensity of the probe for detecting bacteria of genus Staphylococcus and the probe for detecting Gram-positive bacteria, which are targets for detection of Sa, was high and detected. On the other hand, when Ec and Sa were cultured in an NIPAM polymer culture medium, the fluorescence intensity of the probe for detecting bacteria of the genus Staphylococcus and the probe for detecting Gram-positive bacteria was found to be high as in the case of culturing only Sa. It was confirmed that a probe for detecting Gram-negative bacteria, which is a target for detection of Ec, was also detected, so it was found out that Ec was also detected at the same time. These results indicate that simultaneous detection of multiple bacteria, whose growth is inhibited in liquid culture media, is possible by culturing in NIPAM polymer media.

### INDUSTRIAL APPLICABILITY

According to the present disclosure, when target microorganisms are detected in a microorganism-containing sample, ease of handling like liquid culture media is maintained, and even when multiple bacterial species are present in the sample, each bacterial species is allowed to grow sufficiently by suppressing growth inhibition due to the influence of other bacterial species, thereby improving the detection accuracy of the target microorganisms. This technology improves the detection accuracy of target microorganisms from microorganism-containing samples, such as body-derived samples and environmental materials, in which multiple bacterial species are present, thereby contributing to health condition management and environmental analyses.

## Claims

1. A method for detecting microorganisms comprising:
a) inoculating a microorganism-containing sample into a culture medium for microorganisms that contains an environment-responsive polymer that can cause a solution to transition in a thermoreversible manner between a fluidized state and a non-fluidized state depending on a solution environment;
b) culturing microorganisms while maintaining the culture medium in a non-fluidizing environment;
c) adjusting the culture medium to a fluidizing environment after the culturing of the microorganisms;
d) recovering the cultured microorganisms from the culture medium; and
e) detecting target microorganisms in the recovered microorganisms.

2. The method according to claim 1, wherein
the environment-responsive polymer is a thermosensitive polymer, the culture medium for microorganisms is brought into the fluidized state in a thermoreversible manner at temperatures below a transition temperature, and is brought into the non-fluidized state in a thermoreversible manner at temperatures above the transition temperature,
the step b) is performed by maintaining the culture medium at a temperature at which the culture medium becomes non-fluidized, and
the step c) is performed by adjusting the culture medium to a temperature at which the culture medium becomes fluidized.

3. The method according to claim 2, wherein the step a) is performed by immersing a solid substrate on which the microorganisms are captured, in the culture medium that has been adjusted to a temperature at which the culture medium becomes fluidized.

4. The method according to claim 1 or 2, wherein the step e) is performed by detecting nucleic acids, proteins, or components other than nucleic acids and proteins specific to the target microorganisms in the recovered microorganisms.

5. The method according to claim 3, wherein the step e) is performed by:
extracting nucleic acids from the recovered microorganisms; and
detecting a nucleotide sequence specific to the target microorganisms in the extracted nucleic acids.

6. The method according to claim 2, wherein
the step b) is performed at a temperature at least 1°C and at most 40°C above the transition temperature, and
the step c) is performed at a temperature at least 1°C and at most 40°C below the transition temperature.

7. The method according to claim 2, wherein
the step b) is performed at a temperature of 10°C or higher and 60°C or lower, and
the step c) is performed at a temperature of 1°C or higher and 40°C or lower.

8. The method according to claim 2, wherein the transition temperature is 1°C or higher and 40°C or lower.

9. The method according to claim 2, wherein the thermosensitive polymer is readily water-soluble in a thermoreversible manner at temperatures below the transition temperature, and is poorly water-soluble in a thermoreversible manner at temperatures above the transition temperature.

10. The method according to claim 8, wherein the environment-responsive polymer is:
a thermosensitive polymer selected from a group consisting of any one of poly(N-isopropylacrylamide) (PNIPAM), polyacrylamide, polymethacrylate, polycyclodextran, synthetic elastin polymers, amphiphilic polymers with multidentate chelating ability, hydrogels, hyaluronic acid complexes, natural hydrogels, synthetic hydrogels, silicone, polyurethane, polysulfone, cellulose, polyethylene, polypropylene, polyamide, polyimide, polyester, polytetrafluoroethylene, polyvinyl chloride, epoxy resin, phenolic resin, neoprene, and polyisoprene, as well as any combination thereof; or
a pH-sensitive polymer selected from a group consisting of any one of copolymers of N-isopropylacrylamide (NIPAM) and acrylic acid, propylacrylic acid, or 2-carboxyisopropylacrylamide, as well as any combination thereof.

11. A system for detecting microorganisms comprising:
A) a culture medium environment adjuster configured to:
adjust an environment of a culture medium for microorganisms containing an environment-responsive polymer to a fluidizing environment when a sample containing microorganisms is inoculated;
maintain the environment of the culture medium in a non-fluidizing environment while the microorganisms are being cultured; and
adjust the environment of the culture medium to a fluidizing environment when the cultured microorganisms are recovered;
B) a microorganism recoverer configured to recover the cultured microorganisms from the culture medium; and
C) a nucleic acid extractor configured to extract nucleic acids from the recovered microorganisms.

12. The system according to claim 11, wherein
A) the culture medium environment adjuster is configured to:
adjust a temperature of a culture medium for microorganisms containing a thermosensitive polymer to a fluidizing temperature when a sample containing microorganisms is inoculated;
maintain the temperature of the culture medium at a non-fluidizing temperature while the microorganisms are being cultured; and
adjust the temperature of the culture medium to a fluidizing temperature when the cultured microorganisms are recovered.

13. The system according to claim 11 or 12, further comprising:
D) a nucleic acid detector configured to detect a nucleotide sequence specific to target microorganisms in the nucleic acids extracted from the microorganisms.

14. A kit for detecting microorganisms, comprising:
i) the system according to claim 11 or 12; and
ii) any one of the following ii-1) to ii-3):
ii-1) an environment-responsive polymer that, when dissolved in a culture medium for microorganisms, can cause a solution to transition in a thermoreversible manner between a fluidized state and a non-fluidized state depending on a solution environment;
ii-2) a culture medium component composition containing a thermosensitive polymer and a culture medium component for microorganisms; or
ii-3) a culture medium for microorganisms containing a thermosensitive polymer.

15. The kit according to claim 14, further comprising at least one or more of the following:
iii) a sheet substrate for capturing microorganisms;
iv) a culture vessel capable of being installed in the system, the culture vessel containing the culture medium and the sheet substrate; and
v) a probe or primer for detecting a nucleotide sequence specific to target microorganisms.
